# EUROPÄISCHE PATENTANMELDUNG

(11) **EP 2 105 485 A1**
(43) Veröffentlichungstag der Anmeldung: **30.09.2009**
(21) Anmeldenummer: 08153259.0
(22) Anmeldetag: 26.03.2008
(51) Int. Cl.: C09J 163/00, C09J 175/00, G01N 31/22, G01N 33/44

(54) **Zweikomponentige Zusammensetzung mit Mischkontroll-Mittel**

(71) Anmelder: Sika Technology AG, 6340 Baar (CH)
(72) Erfinder: HUCK, Wolf-Rüdiger, 8044 Zürich (CH); TRAN, Linh, 2740 Skovlunde (DK); HAUFE, Markus, 8048 Zürich (CH)
(74) Vertreter: Sika Patent Attorneys

(57) **Zusammenfassung**

Die vorliegende Erfindung betrifft zweikomponentige Zusammensetzungen enthaltend eine lumineszierende Substanz, welche es erlauben auf einfache Art und Weise zu überprüfen, ob die beide Komponenten vorhanden, bzw. ob die zwei Komponenten gemischt wurden, bzw. wie gut die Mischung durchgeführt wurde. Dies ist möglich, ohne dass die Zusammensetzung unter Betrachtung bei Tageslicht visuell negativ verändert wird. Die Zusammensetzungen werden insbesondere für die industrielle Fertigung eingesetzt und erlauben es die Qualität dieses Prozesses stark zu erhöhen.

## Beschreibung

### Technisches Gebiet

Die Erfindung betrifft das Gebiet der zweikomponentigen Zusammensetzungen, insbesondere deren Einsatz in industriellen Prozessen.

### Stand der Technik

Zweikomponentige Zusammensetzungen härten beim Mischen der zwei Komponenten aus. Aufgrund der schnellen Aushärtung einer Vielzahl von zweikomponentigen Zusammensetzungen finden sie in industriellen Prozessen breite Verwendungen. Für die Aushärtung von zweikomponentigen Zusammensetzungen, insbesondere von Klebstoffen, ist eine gute Mischung der zwei Komponenten sehr wichtig. Schlechtes Vermischen, sowie Dosierfehler, können stark negative Auswirkungen auf das Aushärten und somit auf die mechanischen Eigenschaften der ausgehärteten Zusammensetzung haben. Es ist somit für eine verlässliche Qualität sehr wichtig, die Güte der Vermischung sowie die Dosiergenauigkeit zu gewährleisten. Mischfehler sind auf verschiedene Ursachen zurückzuführen. Einerseits ist die Güte der Mischung von der Konsistenz der zwei Komponenten abhängig, andererseits ist sie sehr abhängig von den verwendeten Mischgeräten sowie der Mischzeit. Um beurteilen zu können, ob wirklich beide Komponenten vorhanden sind und wie gut sie miteinander gemischt werden, werden die zwei Komponenten vielfach mit zwei unterschiedlichen Farben eingefärbt. Die Farben werden hierbei üblicherweise derart gewählt, dass sich die Mischfarbe stark von den nicht gemischten Farben unterscheidet. So werden beispielsweise die Farbkombinationen weiss/schwarz, weiss/rot, weiss/blau oder blau/gelb für diesen Zweck verwendet. Das Auftreten von Farbunterschieden oder -Streifen lässt beispielsweise darauf schliessen, dass zuwenig lang gerührt wurde oder dass in einem Statikmischer zu wenig Mischelemente verwendet wurden. Dieses visuelle Beurteilungsverfahren ist aber einerseits sehr unempfindlich und andererseits lässt sich nur eine kleine Anzahl von Farbkombinationen unter Verwendung dieser Methode realisieren. Bei Abdichtungen oder sichtbaren Verklebungen, wie sie beim Verkleben von transparenten Materialien oder dekorativen Anwendungen vielfach auftreten, ist eine derartige Limitierung der Farbe der gemischten Zusammensetzung von grossem Nachteil. Insbesondere sind farblose oder helle Zusammensetzungen somit nicht oder nur sehr schlecht realisierbar.

WO 2007/113287 A1 offenbart eine Methode bei der Infrarotthermografie verwendet wird um die Mischgüte zu ermitteln. Diese Methode bedarf jedoch einer teuren Infrarot-Kamera.

### Darstellung der Erfindung

Die Aufgabe der vorliegenden Erfindung ist es daher, zweikomponentige Zusammensetzungen zur Verfügung zu stellen, welche es auf einfache und billige Art erlauben, die Mischung, bzw. die Mischgüte, der zwei Komponenten zu überprüfen.

Diese Aufgabe wird durch eine Zusammensetzung gemäss Anspruch 1 gelöst. Dadurch wird es möglich, eine derartige Überprüfung durchzuführen, ohne dass die resultierende Zusammensetzung vor oder nach Aushärtung bei Tageslicht sichtbar verändert wird. Weiterhin werden durch den Zusatz der lumineszierenden Substanz die mechanischen Eigenschaften oder Haftungen nicht nachtteilig verändert. Insbesondere lässt sich einerseits grundsätzlich die Anwesenheit derjenigen Komponente, welche die lumineszierende Substanz enthält, nachweisen. Dies ist auch dann der Fall, wenn die Zusammensetzung farblos, weiss, schwarz oder farbig ist, bzw. Schwarzpigmente wie Russ enthält. Insbesondere kann die Mischbarkeit von zwei gleicher Farbe, oder lediglich schwach kontrastierenden Farben beurteilt. Es lässt sich somit zweifelsfrei feststellen, ob diese Komponente zugemischt wird oder nicht. Darüber hinaus lassen sich aber aufgrund bekannter Konzentration der lumineszierenden Substanz in der jeweiligen Komponente und unter Mithilfe einer entsprechenden Eichgerade auch quantitative Aussagen über die Menge der zudosierten Komponente und damit über das Mischungsverhältnis machen und gegebenenfalls das Mischverhältnis korrigieren. Schliesslich kann mit Hilfe einer Bildanalyse Information über die Mischgüte erhalten werden, was wiederum Anlass zur Korrektur, wie Auswechseln des Mischers, führen kann. Somit kann dank vorliegender Erfindung einer Steigerung der Qualität im Verarbeiten von zweikomponentigen Zusammensetzungen erfolgen.

Weitere Aspekte der Erfindung sind Gegenstand weiterer unabhängiger Ansprüche. Besonders bevorzugte Ausführungsformen der Erfindung sind Gegenstand der abhängigen Ansprüche.

### Wege zur Ausführung der Erfindung

Die vorliegende Erfindung betrifft zweikomponentige Zusammensetzungen, welche aus einer ersten Komponenten **K1** und einer zweiten Komponente **K2** bestehen.

Die Komponente **K1** enthält hierbei mindestens eine härtbare Verbindung **HV.**

Die Komponente **K2** enthält hierbei mindestens eine Verbindung **X,** welche mit der härtbaren Verbindung **HV** der Komponente **K1** reaktionsfähig ist oder welche die Polymerisation der härtbaren Verbindung **HV** der Komponente **K1** auslöst.

Zudem enthält eine der zwei Komponenten **K1** oder **K2** mindestens eine lumineszierende Substanz **L.**

Der Begriff "Polymer" umfasst im vorliegenden Dokument einerseits ein Kollektiv von chemisch einheitlichen, sich aber in Bezug auf Polymerisationsgrad, Molmasse und Kettenlänge unterscheidenden Makromolekülen, das durch eine Polyreaktion (Polymerisation, Polyaddition, Polykondensation) hergestellt wurde. Der Begriff umfasst andererseits auch Derivate eines solchen Kollektivs von Makromolekülen aus Polyreaktionen, Verbindungen also, die durch Umsetzungen, wie beispielsweise Additionen oder Substitutionen, von funktionellen Gruppen an vorgegebenen Makromolekülen erhalten wurden und die chemisch einheitlich oder chemisch uneinheitlich sein können. Der Begriff umfasst im Weiteren auch sogenannte Prepolymere, das heisst reaktive oligomere Voraddukte, deren funktionelle Gruppen am Aufbau von Makromolekülen beteiligt sind.

Der Begriff "Polyurethanpolymer" umfasst sämtliche Polymere, welche nach dem sogenannten Diisocyanat-Polyadditions-Verfahren hergestellt werden. Dies schliesst auch solche Polymere ein, die nahezu oder gänzlich frei sind von Urethangruppen. Beispiele für Polyurethanpolymere sind Polyether-Polyurethane, Polyester-Polyurethane, Polyether-Polyharnstoffe, Polyharnstoffe, Polyester-Polyharnstoffe, Polyisocyanurate und Polycarbodiimide.

Als "Raumtemperatur" wird eine Temperatur von 25 °C bezeichnet.

Mit "Poly" beginnende Substanznamen wie Polyisocyanat, Polyol, Polymercaptan, Polyepoxid oder Polyamin bezeichnen im vorliegenden Dokument Substanzen, die formal zwei oder mehr der in ihrem Namen vorkommenden funktionellen Gruppen pro Molekül enthalten.

Die in Klammern vorhandene Vorsilbe "Poly" in (Poly)Aminoalkohol, bzw. (Poly)Aminomercaptan, bzw. (Poly)Mercaptoalkohol, ist derart zu verstehen, dass in demselben Molekül mindestens eine Amino- und mindestens eine Hydroxygruppe, bzw. mindestens eine Amino- und mindestens eine Mercaptogruppe, bzw. mindestens eine Mercapto- und mindestens eine Hydroxygruppe, vorhanden ist.

Als "(Meth)acrylate" sind Ester der Acrylsäure oder Methacrylsäure zu verstehen.

Der Begriff "primäre Aminogruppe" bezeichnet im vorliegenden Dokument eine NH₂-Gruppe, die an einen organischen Rest gebunden ist, während der Begriff "sekundäre Aminogruppe" eine NH-Gruppe bezeichnet, die an zwei organische Reste, welche auch gemeinsam Teil eines Rings sein können, gebunden ist.

Als "aliphatische Aminogruppe" wird eine Aminogruppe bezeichnet, die an einen aliphatischen, cycloaliphatischen oder arylaliphatischen Rest gebunden ist. Sie unterscheidet sich damit von einer "aromatischen Aminogruppe", welche direkt an einen aromatischen oder heteroaromatischen Rest gebunden ist, wie beispielsweise in Anilin oder 2-Aminopyridin.

In einer ersten, besonders bevorzugten, Ausführungsform ist die härtbare Verbindung **HV** ein Polyepoxid und die Verbindung **X** ist ausgewählt aus der Gruppe bestehend aus Polyaminen, Polymercaptanen, Polyamidoaminen, (Poly)Aminoalkoholen, (Poly)Mercaptoalkoholen und (Poly)Aminomer-captanen.

Als Polyepoxid gelten insbesondere Polyglycidylether. Bevorzugt werden einerseits sogenannte Epoxidharze und anderseits sogenannte Epoxid-Reaktivverdünner.

Als Epoxidharze gelten Epoxid-Flüssigharze oder ein Epoxid-Festharze mit durchschnittlich mehr als einer Epoxidgruppe pro Molekül. Der Begriff "Epoxid-Festharz" ist dem Epoxid-Fachmann bestens bekannt und wird im Gegensatz zu "Epoxid-Flüssigharzen" verwendet. Die Glastemperatur von Festharzen liegt über Raumtemperatur, d.h. sie lassen sich bei Raumtemperatur zu schüttfähigen Partikel zerkleinern. Bevorzugte Epoxid-Festharze weisen die Formel (I) auf.

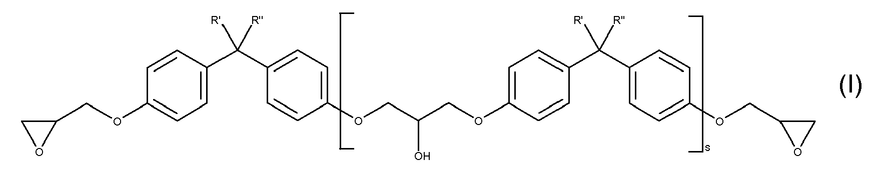

Hierbei stehen die Substituenten R' und R" unabhängig voneinander entweder für H oder CH₃. Der Term "unabhängig voneinander" in der Definition von Gruppen und Resten bedeutet in diesem Dokument jeweils, dass mehrere vorkommende aber in den Formeln gleich bezeichnete Gruppen jeweils unterschiedliche Bedeutungen aufweisen können.

Weiterhin steht der Index s für einen Wert von > 1.5, insbesondere von 2 bis 12.

Derartige Epoxid-Festharze sind kommerziell erhältlich beispielsweise von Dow oder Huntsman oder Hexion.

Verbindungen der Formel (I) mit einem Index s zwischen 1 und 1.5 werden vom Fachmann als Semisolid-Epoxidharze bezeichnet. Für die hier vorliegende Erfindung werden sie ebenfalls als Festharze betrachtet. Bevorzugt sind jedoch Epoxidharze im engeren Sinn, d.h. wo der Index s einen Wert von > 1.5 aufweist.

Bevorzugte Epoxid-Flüssigharze weisen die Formel (II) auf 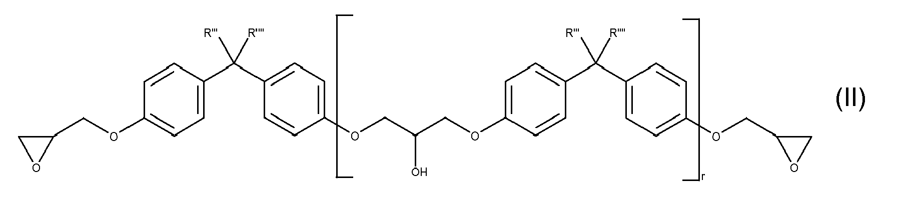 Hierbei stehen die Substituenten R''' und R'''' unabhängig voneinander entweder für H oder CH₃. Weiterhin steht der Index r für einen Wert von 0 bis 1. Bevorzugt steht r für einen Wert von kleiner als 0.2.
Es handelt sich somit vorzugsweise um Diglycidylether von Bisphenol-A (DGEBA), von Bisphenol-F sowie von Bisphenol-A/F (Die Bezeichnung 'A/F' verweist hierbei auf eine Mischung von Aceton mit Formaldehyd, welche als Edukt bei dessen Herstellung verwendet wird). Solche Flüssigharze sind beispielsweise als Araldite® GY 250, Araldite® PY 304, Araldite® GY 282 (Huntsman, bzw. Hexion) oder D.E.R.™ 331 oder D.E.R.™ 330 (Dow) oder Epikote 828 (Hexion) erhältlich.

Bevorzugt stellt das Polyepoxid ein Epoxid-Flüssigharz der Formel (II) dar. In einer noch mehr bevorzugten Ausführungsform enthält die hitzehärtende Epoxidharzzusammensetzung sowohl mindestens ein Epoxid-Flüssigharz der Formel (II) als auch mindestens ein Epoxid-Festharz der Formel (I).

Als Epoxid-Reaktivverdünner sind besonders Glycidylether von tri- oder polyfunktionellen, gesättigten oder ungesättigten, verzweigten oder unverzweigten, zyklischen oder offenkettigen Alkoholen wie epoxidiertes Rhizinusöl, epoxidiertes Trimethylolpropan, epoxidiertes Pentaerythrol oder Polyglycidylether von aliphatischen Polyolen wie Sorbitol, Glycerin oder Trimethylolpropan, geeignet.

Weiterhin geeignet als Polyepoxide sind solche, welche die folgende Formel aufweisen 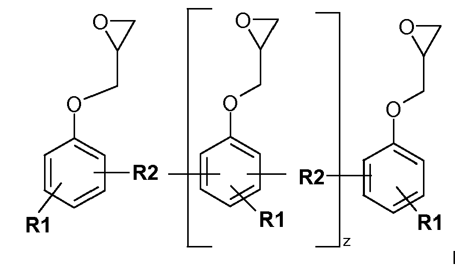 mit **R2**= 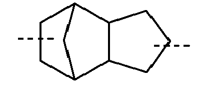 oder CH₂, **R1** =
H oder Methyl und z = 0 bis 7.
Insbesondere handelt es sich hierbei um Phenol- oder Kresol- Novolake (**R2** = CH₂).

Solche Polyepoxide sind unter dem Handelnamen EPN oder ECN sowie Tactix®556 von Huntsman kommerziell erhältlich, verschiedene EPN und ECN -Typen werden auch beispielsweise von Huntsman als Dispersion in Wasser angeboten.

Als Polyamine sind insbesondere geeignet
- Aliphatische Polyamine wie Ethylendiamin, 1,2- und 1,3-Propandiamin, 2-Methyl-1,2-propandiamin, 2,2-Dimethyl-1,3-propandiamin, 1,3- und 1,4-Butandiamin, 1,3- und 1,5-Pentandiamin, 1,5-Diamino-2-methylpentan (MPMD), 1,6-Hexandiamin, 2,2,4- und 2,4,4-Trimethylhexamethylendiamin, 1,7-Heptandiamin, 1,8-Octandiamin, 4-Aminomethyl-1,8-octandiamin, Methyl-bis-(3-aminopropyl)amin, 1,3-Diaminopentan (DAMP), 2,5-Dimethyl-1,6-hexamethylendiamin, Diethylentriamin (DETA), Triethylentetramin (3,6-Diaza-octamethylendiamin) (TETA), Tetraethylenpentamin (TEPA), Pentamethylenhexamin, Dipropylentriamin, Tripropylentetramin, Tetrapropylenpentamin, 4,7-Diaza-decamethylen-1,10-diamin sowie Mischungen der vorgenannten Polyamine.
- cycloaliphatische Polyamine wie 1,3- und 1,4-Diaminocyclohexan, 1,2-Diaminocyclohexan (DCH), Bis-(4-aminocyclohexyl)-methan (PACM), Bis-(4-amino-3-methylcyclohexyl)-methan, Bis-(4-amino-3-ethylcyclohexyl)-methan, Bis-(4-amino-3,5-dimethylcyclohexyl)-methan,1-Amino-3-aminomethyl-3,5,5-trimethylcyclohexan (= Isophorondiamin oder IPDA), 2- und 4-Methyl-1,3-diaminocyclohexan, 1,3- und 1,4-Bis-(aminomethyl)cyclohexan, 1,3-2,5(2,6)-Bis-(aminomethyl)-bicyclo[2.2.1]heptan (NBDA, hergestellt von Mitsui Chemicals), 3(4),8(9)-Bis-(aminomethyl)-tricyclo[5.2.1.02,6]decan, 3,9-Bis-(3-aminopropyl)-2,4,8,10-tetraoxaspiro[5.5]undecan, Piperazin, 1-(2-Aminoethyl)piperazin, 1,3- und 1,4-Xylylendiamin, Octahydro-4,7-methano-indene-2,5-diamin, Octahydro-4,7-methano-indene-1,6-diamin, Ethergruppen-haltige aliphatische Polyamine wie Bis-(2-aminoethyl)ether, und höhere Oligomere davon, sowie Mischungen der vorgenannten Polyamine.
- aromatische Amine wie Toluylendiamin, Phenylendiamin, 4,4 -Methylendianilin (MDA) sowie Mischungen der vorgenannten Polyamine.
- Ethergruppen-haltige aliphatische Polyamine wie Bis-(2-aminoethyl)ether, 4,7-Dioxadecan-1,10-diamin, 4,9-Dioxadodecan-1,12-diamin und höhere Oligomere davon, Polyoxyalkylen-Polyamine mit zwei oder drei Aminogruppen, beispielsweise erhältlich unter dem Namen Jeffamine^{®} (von Huntsman Chemicals), unter dem Namen Polyetheramin (von BASF) oder unter dem Namen PC Amine^{®} (von Nitroil), sowie Mischungen der vorgenannten Polyamine. Insbesondere bevorzugt sind Jeffamine®, wie sie unter der D-Linie und ED-Linie von Huntsman Chemicals angeboten werden, wie zum Besipiel Jeffamine® D-230, Jeffamine® D-400, Jeffamine® D-2000, Jeffamine® D-4000, Jeffamine® ED-600, Jeffamine® ED-900, Jeffamine® ED-2003 oder Jeffamine® EDR-148.

Als Polymercaptane sind beispielsweise Polymercaptoacetate von Polyolen geeignet. Es handelt sich hierbei insbesondere um Polymercaptoacetate der folgenden Polyole:
- Polyoxyalkylenpolyole, auch Polyetherpolyole genannt, welche das Polymerisationsprodukt von Ethylenoxid, 1,2-Propylenoxid, 1,2- oder 2,3-Butylenoxid, Oxetan, Tetrahydrofuran oder Mischungen davon sind, gegebenenfalls polymerisiert mit Hilfe eines Startermoleküls mit zwei oder drei aktiven H-Atomen wie beispielsweise Wasser oder Verbindungen mit zwei oder drei OH-Gruppen. Eingesetzt werden können sowohl Polyoxyalkylenpolyole, die einen niedrigen Ungesättigtheitsgrad aufweisen (gemessen nach ASTM D-2849-69 und angegeben in Milliequivalent Ungesättigtheit pro Gramm Polyol (mEq/g)), hergestellt beispielsweise mit Hilfe von sogenannten Double Metal Cyanide Complex Katalysatoren (kurz DMC-Katalysatoren), als auch Polyoxyalkylenpolyole mit einem höheren Ungesättigtheitsgrad, hergestellt beispielsweise mit Hilfe von anionischen Katalysatoren wie NaOH, KOH oder Alkalialkoholaten. Besonders geeignet sind Polyoxypropylendiole und -triole mit einem Ungesättigtheitsgrad tiefer als 0.02 mEq/g und mit einem Molekulargewicht im Bereich von 300 - 20'000 Dalton, Polyoxybutylendiole und -triole, Polyoxypropylendiole und - triole mit einem Molekulargewicht von 400 - 8'000 Dalton, sowie sogenannte "EO-endcapped" (ethylene oxide-endcapped) Polyoxypropylendiole oder -triole. Letztere sind spezielle Polyoxypropylenpolyoxyethylenpolyole, die beispielsweise dadurch erhalten werden, dass reine Polyoxypropylenpolyole nach Abschluss der Polypropoxylierung mit Ethylenoxid alkoxyliert werden und dadurch primäre Hydroxylgruppen aufweisen.
- Hydroxyterminierte Polybutadienpolyole, wie beispielsweise solche, die durch Polymerisation von 1,3-Butadien und Allylalkohol oder durch Oxidation von Polybutadien hergestellt werden, sowie deren Hydrierungsprodukte;
- Styrol-Acrylnitril gepfropfte Polyetherpolyole, wie sie beispielsweise von Elastogran unter dem Namen Lupranol® geliefert werden;
- Polyesterpolyole, hergestellt beispielsweise aus zwei- bis dreiwertigen Alkoholen wie beispielsweise 1,2-Ethandiol, Diethylenglykol, 1,2-Propandiol, Dipropylenglykol, 1,4-Butandiol, 1,5-Pentandiol, 1,6-Hexandiol, Neopentylglykol, Glycerin, 1,1,1-Trimethylolpropan oder Mischungen der vorgenannten Alkohole mit organischen Dicarbonsäuren oder deren Anhydriden oder Estern wie beispielsweise Bernsteinsäure, Glutarsäure, Adipinsäure, Korksäure, Sebacinsäure, Dodecandicarbonsäure, Maleinsäure, Fumarsäure, Phthalsäure, Isophthalsäure, Terephthalsäure und Hexahydrophthalsäure oder Mischungen der vorgenannten Säuren, sowie Polyesterpolyole aus Lactonen wie beispielsweise ε-Caprolacton;
- Polycarbonatpolyole, wie sie durch Umsetzung beispielsweise der oben genannten - zum Aufbau der Polyesterpolyole eingesetzten - Alkohole mit Dialkylcarbonaten, Diarylcarbonaten oder Phosgen zugänglich sind;
- 1,2-Ethandiol, Diethylenglykol, 1,2-Propandiol, Dipropylenglykol, 1,4-Butandiol, 1,5-Pentandiol, 1,6-Hexandiol, 1,7-Heptandiol, Octandiol, Nonandiol, Decandiol, Neopentylglykol, Pentaerythrit (= 2,2-Bis-hydroxymethyl-1,3-propandiol), Dipentaerythrit (= 3-(3-Hydroxy-2,2-bis-hydroxymethyl-propoxy)-2,2-bis-hydroxymethyl-propan-1-ol), Glycerin (= 1,2,3-Propantriol), Trimethylolpropan (= 2-Ethyl-2-(hydroxymethyl)-1,3-propandiol), Trimethylolethan (= 2-(hydroxymethyl)-2-Methyl-1,3-propandiol), Di(trimethylolpropan) (= 3-(2,2-Bis-hydroxymethyl-butoxy)-2-ethyl-2-hydroxymethyl-propan-1-ol), Di(trimethylolethan) (= 3-(3-Hydroxy-2-hydroxymethyl-2-methyl-propoxy)-2-hydroxymethyl-2-methyl-propan-1-ol), Diglycerin (= Bis-(2,3-dihydroxypropyl)-ether);
- Polyole, wie sie durch Reduktion von dimerisierten Fettsäuren enthalten werden.

Insbesondere bevorzugt sind Glycol-dimercaptoacetat, Trimethylolpropan-trimercaptoacetat und Butandiol-dimercaptoacetat.

Als meist bevorzugte Polymercaptane gelten Dimercaptane der Formel (III).

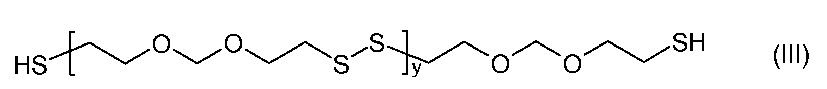

Hierbei steht y für einen Wert von 1 bis 45, insbesondere von 5 bis 23. Die bevorzugten Molekulargewichte sind zwischen 800 und 7500 g/mol, insbesondere zwischen 1000 und 4000 g/mol.

Derartige Polymercaptane sind kommerziell erhältlich unter der Thiokol® LP-Reihe von Toray Fine Chemicals Co.

Als (Poly)Aminoalkohole sind insbesondere geeignet solche, welche ausgewählt sind aus der Gruppe bestehend aus Aminoethanol, 2-Aminopropanol, 3-Aminopropanol, 2-Amino-2,2-dimethylethanol, 2-Amino-1-methyl-ethanol und 2-Amino-propan-2-ol.

Als (Poly)Mercaptoalkohole sind insbesondere geeignet solche, welche ausgewählt sind aus der Gruppe bestehend aus Thioethanol, 2-Thiopropanol, 3-Thiopropanol und Thioglycerin.

Als (Poly)Aminomercaptane sind insbesondere geeignet sind Aminoethanthiol und Aminodithiopropan.

Das Verhältnis des Polyepoxids zur Verbindung **X** ist vorzugsweise derart gewählt, dass in der Zusammensetzung die Anzahl der Epoxidgruppen der Komponente **K1** im Wesentlichen stöchiometrisch zu den Amino- bzw. Mercaptogruppen der Komponente **K2** ist. Es gilt hierbei, dass eine primäre Aminogruppe zweimal mit einer Epoxidgruppe zu reagieren vermag. Unter "im Wesentlichen" ist hier eine Abweichung von bis zu ± 20 %, insbesondere bis zu ± 10 %, bevorzugt bis zu ± 5 %, gegenüber dem stöchiometrischen Verhältnis zu verstehen.

In einer zweiten Ausführungsform ist die härtbare Verbindung **HV** ein Polyisocyanat und die Verbindung **X** ist ausgewählt aus der Gruppe bestehend aus Wasser, Polyolen, Polyaminen, Polymercaptanen, Polyamidoaminen, (Poly)Aminoalkoholen, (Poly)Mercaptoalkoholen und (Poly)Aminomercap-tanen.

Als Polyisocyanat sind in einer Ausführungsform insbesondere geeignet sind aromatische oder aliphatische Polyisocyanate, insbesondere die Diisocyanate.

Als "aromatisches Isocyanat" wird eine organische Verbindung bezeichnet, welche ausschliesslich aromatische Isocyanatgruppen aufweist. Als "aromatisch" wird eine Isocyanatgruppe bezeichnet, die an einen aromatischen oder heteroaromatischen Rest gebunden ist. Als "aliphatisches Isocyanat" wird eine organische Verbindung bezeichnet, die aliphatische Isocyanatgruppen enthält. Als "aliphatisch" wird eine Isocyanatgruppe bezeichnet, die an einen aliphatischen, cycloaliphatischen oder arylaliphatischen Rest gebunden ist.

Als aromatische Polyisocyanate eignen sich beispielsweise monomere Di- oder Triisocyanate wie 2,4- und 2,6-Toluylendiisocyanat und beliebige Gemische dieser Isomeren (TDI), 4,4'-, 2,4'- und 2,2'-Diphenylmethandiisocyanat und beliebige Gemische dieser Isomeren (MDI), Gemische aus MDI und MDI-Homologen (polymeres MDI oder PMDI), 1,3- und 1,4-Phenylendiisocyanat, 2,3,5,6-Tetramethyl-1,4-diisocyanatobenzol, Naphthalin-1,5-diisocyanat (NDI), 3,3'-Dimethyl-4,4'-diisocyanatodiphenyl (TODI), Dianisidindiisocyanat (DADI), 1,3,5-Tris-(isocyanatomethyl)-benzol, Tris-(4-isocyanatophenyl)-methan, Tris-(4-isocyanatophenyl)-thiophosphat, Oligomere und Polymere der vorgenannten Isocyanate, sowie beliebige Mischungen der vorgenannten Isocyanate. Bevorzugt sind MDI und TDI.

Als aliphatische Polyisocyanate eignen sich beispielsweise monomere Di- oder Triisocyanate wie 1,4-Tetramethylendiisocyanat, 2-Methylpenta-methylen-1,5-diisocyanat, 1,6-Hexamethylendiisocyanat (HDI), 2,2,4- und 2,4,4-Trimethyl-1,6-hexamethylendiisocyanat (TMDI), 1,10-Decamethylendiisocyanat, 1,12-Dodecamethylendiisocyanat, Lysin- und Lysinesterdiisocyanat, Cyclohexan-1,3- und -1,4-diisocyanat, 1-Methyl-2,4- und -2,6-diisocyanatocyclohexan und beliebige Gemische dieser Isomeren (HTDI oder H₆TDI), 1-Isocyanato-3,3,5-trimethyl-5-isocyanatomethyl-cyclohexan (=Isophorondiisocyanat oder IPDI), Perhydro-2,4'- und -4,4'-diphenylmethandiisocyanat (HMDI oder H₁₂MDI), 1,4-Diisocyanato-2,2,6-trimethylcyclohexan (TMCDI), 1,3- und 1,4-Bis-(isocyanatomethyl)-cyclohexan, m- und p-Xylylendiisocyanat (m- und p-XDI), m- und p-Tetramethyl-1,3- und -1,4-xylylendiisocyanat (m- und p-TMXDI), Bis-(1-Isocyanato-1-methylethyl)-naphthalin, Dimer- und Trimerfettsäureisocyanate wie 3,6-Bis-(9-isocyanatononyl)-4,5-di-(1-heptenyl)-cyclohexen (Dimeryldiisocyanat), α,α,α',α',α",α"-Hexamethyl-1,3,5-mesitylentriiso-cyanat, Oligomere und Polymere der vorgenannten Isocyanate, sowie beliebige Mischungen der vorgenannten Isocyanate. Bevorzugt sind HDI und IPDI.

Als Polyisocyanate geeignet sind weiterhin Polyisocyanate in der Form eines monomeren Di- oder Triisocyanates oder eines Oligomeren eines monomeren Diisocyanates, wobei als monomeres Di- oder Triisocyanat beispielsweise die vorgenannten aromatischen und aliphatischen Di- und Triisocyanate geeignet sind.

Als Oligomer eines monomeren Diisocyanates geeignet sind insbesondere die Oligomere von HDI, IPDI und TDI. Solche Oligomere stellen in der Praxis üblicherweise Gemische von Substanzen mit unterschiedlichen Oligomerisationsgraden und/oder chemischen Strukturen dar. Vorzugsweise weisen sie eine mittlere NCO-Funktionalität von 2.1 bis 4.0 auf und enthalten insbesondere Isocyanurat-, Iminooxadiazindion-, Uretdion-, Urethan-, Biuret-, Allophanat-, Carbodiimid-, Uretonimin- oder Oxadiazintrion-Gruppen. Bevorzugt weisen sie einen niedrigen Gehalt an monomeren Diisocyanaten auf. Kommerziell erhältliche Typen sind insbesondere HDI-Biurete, beispielsweise Desmodur^{®} N 100 und Desmodur^{®} N 3200 (von Bayer), Tolonate^{®} HDB und Tolonate^{®} HDB-LV (von Rhodia) sowie Duranate^{®} 24A-100 (von Asahi Kasei); HDI-Isocyanurate, beispielsweise Desmodur^{®} N 3300, Desmodur^{®} N 3600 und Desmodur^{®} N 3790 BA (von Bayer), Tolonate^{®} HDT, Tolonate^{®} HDT-LV und Tolonate^{®} HDT-LV2 (von Rhodia), Duranate^{®} TPA-100 und Duranate^{®} THA-100 (von Asahi Kasei) sowie Coronate^{®} HX (von Nippon Polyurethane); HDI-Uretdione, beispielsweise Desmodur^{®} N 3400 (von Bayer); HDI-Iminooxadiazindione, beispielsweise Desmodur^{®} XP 2410 (von Bayer); HDI-Allophanate, beispielsweise Desmodur^{®} VP LS 2102 (von Bayer); IPDI-Isocyanurate, beispielsweise Desmodur^{®} Z 4470 (von Bayer) und Vestanat^{®} T1890/100 (von Evonik); TDI-Oligomere, beispielsweise Desmodur^{®} IL (von Bayer); sowie gemischte Isocyanurate auf Basis TDI / HDI, zum Beispiel Desmodur^{®} HL (von Bayer).

Weiterhin als Polyisocyanat geeignet sind Polyisocyanate in der Form einer bei Raumtemperatur flüssigen Form von MDI oder einer Form von polymerem MDI (PMDI).

Bei Raumtemperatur flüssige Formen von MDI (sogenanntes "modifiziertes MDI") stellen Gemische von MDI mit MDI-Derivaten, wie beispielsweise MDI-Carbodiimiden, MDI-Uretoniminen oder MDI-Urethanen, dar. Kommerziell erhältliche Typen von modifiziertem MDI sind beispielsweise Desmodur^{®} CD, Desmodur^{®} PF und Desmodur^{®} PC (von Bayer), Lupranat^{®} MM 103 (von BASF), Isonate^{®} M 143 (von Dow) sowie Suprasec^{®} 2020 und Suprasec^{®} 2388 (von Huntsman).

Als polymeres MDI oder PMDI werden Gemische aus MDI und MDI-Homologen bezeichnet. Kommerziell erhältliche Typen von PMDI sind beispielsweise Desmodur^{®} VL, Desmodur^{®} VL 50, Desmodur^{®} VL R 10, Desmodur^{®} VL R 20 und Desmodur^{®} VKS 20 F (von Bayer), Lupranat^{®} M 10 R und Lupranat^{®} M 20 R (von BASF), Isonate^{®} M 309, Voranate^{®} M 229 und Voranate M^{®} 580 (von Dow) sowie Suprasec^{®} 5025, Suprasec^{®} 2050 und Suprasec^{®} 2487 (von Huntsman).

Als Polyisocyanat sind in einer weiteren Ausführungsform insbesondere Isocyanatgruppen aufweisende Polymere, insbesondere Isocyanatgruppen aufweisende Polyurethanpolymere geeignet, welche sich insbesondere aus der Reaktion der soeben genannten Polyisocyanaten und Polyolen, insbesondere denjenigen Polyolen, welche bereits vorgängig für die erste Ausführungsform beschrieben worden sind, erhalten lassen. Dem Polyurethan-Fachmann für sind die einzuhaltenden Verhältnisse sowie Reaktionsbedingungen für die Herstellung derartiger NCO-Gruppen aufweisenden Polymere bestens bekannt.

Als Polyole, Polyamine, Polymercaptane, Polyamidoamine, (Poly)Aminoalkohole, (Poly)Mercaptoalkohole und (Poly)Aminomercaptane für die Verbindung X besonders geeignet sind jene, welche bereits vorgängig für die erste Ausführungsform beschrieben worden sind.

Das Verhältnis des Polyisocyanats zur Verbindung **X** ist vorzugsweise derart gewählt, dass in der Zusammensetzung die Anzahl der Isocyanatgruppen der Komponente **K1** im Wesentlichen stöchiometrisch zu Wasser oder Hydroxyl- bzw. Wasser-, Amino- oder Mercaptogruppen der Komponente **K2** ist. Es gilt hierbei, dass eine primäre Aminogruppe zweimal mit einer Isocyanatgruppe zu reagieren vermag. Unter "im Wesentlichen" ist hier eine Abweichung von bis zu ± 10 %, insbesondere bis zu ± 5 %, bevorzugt bis zu ± 5 %, gegenüber dem stöchiometrischen Verhältnis zu verstehen.

Da die Isocyanatgruppe jedoch auch mit Luftfeuchtigkeit zu reagieren vermag, ist durchaus auch ein überstöchiometrisches Verhältnis von Anzahl der Isocyanatgruppen der Komponente **K1** zu Wasser oder zu den Hydroxyl- bzw. Wasser-, Amino- oder Mercaptogruppen der Komponente **K2** möglich. Ein derartiges überstöchiometrisches Verhältnis ist insbesondere zwischen 1.01 bis 200. Ist dieses Verhältnis stark überstöchiometrisch, d.h. > 1.5, insbesondere > 2, noch mehr bei > 3, ist die Aushärtungsgeschwindigkeit und Anfangsfestigkeit deutlich geringer als beim stöchiometrischen Umsatz. Dies ist jedoch je nach Verwendung und Anforderungen vielfach nicht nachteilig, zuweilen sogar beabsichtigt. So lassen sich auf elegante Art und Weise bei sehr reaktiven Komponenten **K1** bzw. Verbindungen **X** die Topfzeiten und Anfangsfestigkeiten in einen, für die Applikation gewünschten Bereich verschieben. Es sind demzufolge durchaus auch überstöchiometrische Verhältnisse von mehr als 10, typischerweise jedoch kleiner als 200, insbesondere kleiner als 100, möglich.

In einer dritten Ausführungsform ist die härtbare Verbindung **HV** ein olefinisch ungesättigtes Monomer und/oder Oligomer, welches insbesondere ausgewählt ist aus der Gruppe bestehend aus Styrol, Acrylonitril, (Meth)acrylamide, (Meth)acrylsäure, (Meth)acrylate, Vinylalkohole, Vinylether, Vinylester, und ungesättigte Polyester, bevorzugt ein (Meth)acrylat. Die Verbindung **X** ist hierbei ein Radikalbildner, insbesondere ein Perester oder ein organisches Peroxid oder Hydroperoxid. Bevorzugt ist die Verbindung **X** ein organisches Peroxid. Der Radikalbildner kann jedoch auch andere Natur sein. So sind beispielsweise auch Azo-Verbindungen geeignet, welche durch thermischen Zerfall Radiklae bilden. Als bevorzugte derartige Azo-Verbindung gilt Azo-bis-(isobutyronitril) (AIBN).

Bevorzugt handelt es sich um Ester von (Meth)acrylsäure und einem Alkohol ausgewählt aus der Gruppe bestehend aus Methanol, Ethanol, Propanol, Butanol, Ethylenglycol, Propylenglycol, Butylenglykol, Trimetholpropan, Glycerin, Bisphenol-A, Bisphenol-F, Norboneol, Iso-Norborneol, Trimethylcyclohexanol, Tetrahydrofurfurylalkohol und die Alkoxylierungsprodukte der vorgenannten Alkohole. Falls es sich bei den genannten Alkoholen um Polyole handelt, können die (Meth)acrylate Monoester oder Polyester sein, d.h. unter Umständen kann es vorteilhaft sein, Teilester zu verwenden. Hydroxyethyl(meth)acrylat ist z.B. ein Teilester von Ethylenglycol und (Meth)acrylsäure. Ob (Meth)acrylat auch chemisch über eine Veresterungsreaktion hergestellt wird oder nicht, ist hierbei unerheblich. So kann Hydroxypropyl(meth)acrylat über eine Veresterungsreaktion von Propylenglycol und (Meth)acrylsäure oder durch Propoxylierung von (Meth)acrylsäure hergestellt werden.

Besonders bevorzugt ist das (Meth)acrylat ausgewählt aus der Gruppe bestehend aus 2-Hydroxyethylmethacrylat (HEMA), Hydroxypropylmethacrylat (HPMA), Trimethylcyclohexylmethacrylat (TMCHMA), Cyclohexylmethacrylat (CHMA), Isobornylmethacrylat (IBMA), Tetrahydrofurfurylmethacrylat (THFMA). Meist bevorzugt ist Tetrahydrofurfurylmethacrylat (THFMA).

Als Verbindung **X** ist insbesondere ein organisches Peroxid bevorzugt. Meist bevorzugt ist Dibenzoylperoxid.

Es ist vorteilhaft, wenn die Zusammensetzung in dieser Ausführungsform ein Amin oder Übergangsmetallsalz oder Übergangsmetallkomplex als Katalysator enthält. Die Anwesenheit eines

derartigen Katalysators bewirkt, dass die Radikale bereits bei tieferer Temperatur, insbesondere bei Raumtemperatur, gebildet werden. Geeignete tertiäre Amine sind beispielsweise N,N-Dimethylanilin, N,N-Dimethyl-p-Toluidin, N,N-Diethylanilin, N,N-Diethyltoluidin, N,N-bis(2-hydroxyethyl)-p-Toluidin, N-ethoxylierte p-Toluidine, N-Alkylmorpholine oder Mischungen davon. Besonders geeignete Übergangsmetallsalze oder Übergangsmetallkomplexe sind insbesondere diejenigen der Metalle Cobalt, Mangan, Vanadium und Kupfer.

Als Katalysator werden tertiäre Amine bevorzugt.

Der Anteil des Katalysators an zweikomponentigen Zusammensetzung beträgt vorzugsweise zwischen 0.01 und 10 Gew.-%, insbesondere zwischen 0.1 und 5 Gew.-%.

Da die Verbindung **X** in diesem Falle die Polymerisation des olefinisch ungesättigten Monomers der Komponente **K1** auslöst, und es sich im Gegensatz zur ersten bzw. zweiten Ausführungsform in dieser Ausführungsform nicht um eine chemische Vernetzung von Verbindung **X** und härtbarer Verbindung **HV** handelt, sondern um eine Polymerisationsinitiierung, bzw. Katalyse, der härtbaren Verbindung **HV** der Komponente **K1**, sind in der dritten Ausführungsform lediglich geringe Menge der Verbindung **X** notwendig. Typischerweise ist der Anteil des Radikalbildners am Gewicht der gesamten Zusammensetzung zwischen 0.01 - 15 Gew.-%, insbesondere zwischen 0.5 und 10 Gew.-%.

Weiterhin enthält eine der zwei Komponenten **K1** oder **K2** mindestens eine lumineszierende Substanz **L.** Es kann von Vorteil sein, wenn diese Komponente mehrere lumineszierende Substanzen **L** enthält.

Geeignete lumineszierende Substanzen sind solche, welche bei Bestrahlung mit einer Strahlung bestimmter Wellenlänge eine Strahlung unterschiedlicher Wellenlänge emittieren, insbesondere solche, welche bei Bestrahlung mit UV-Strahlung lumineszieren, bevorzugt solche, die bei Bestrahlung mit Strahlung einer Wellenlänge zwischen 240 und 400 nm lumineszieren. Dem Fachmann sind viele lumineszierende Substanzen bekannt. Derartige lumineszierende Substanzen sind beispielsweise beschrieben in Kirk-Othmer, "Encyclopedia of Chemical Technology", Vol. 11, (John Wiley & Sons, 4th Ed., 1994) auf den Seiten 227 - 241.

Die lumineszierende Substanz ist insbesondere ausgewählt aus der Gruppe bestehend aus Stilben-Derivate, Pyrazoline, Bis(benzoxazol-2-yl)-Derivate und Cumarine.

Insbesondere geeignet sind Substanzen, welche unter den Handelsmarken Uvitex® von Ciba Specialty Chemicals, Lumilux® von Riedelde Haen GmbH, Blankophor® von Bayer Chemicals und Ultraphor® von BASF kommerziell erhältlich sind. Bevorzugt wird Uvitex® OB (Ciba Specialty Chemicals).

Falls die Komponente, in welcher die lumineszierende Verbindung L vorhanden ist, Wasser enthält sind insbesondere jene lumineszierenden Substanzen geeignet, welche unter den Handelsmarken Uvitex® NFW, Uvitex® BFA, Uvitex® MST oder Tinopal® von Ciba Specialty Chemicals und Pyranin® von Bayer Chemicals kommerziell erhältlich sind.

Die einzelnen lumineszierenden Substanzen haben bei Bestrahlung mit einer Strahlung bestimmter Wellenlänge für sich charakteristische Emissionswellenlängen. Die Emissionswellenlänge kann somit ein wichtiges Auswahlkriterium für die lumineszierende Substanz sein.

Die Menge der lumineszierenden Substanz ist sehr abhängig von der Art der lumineszierenden Substanz. Insbesondere wesentlich sind hierbei einerseits die Absorptions- und Emissions-Quantenausbeuten, sowie die Wellenlänge der anregenden, bzw. der emittierenden, bzw. der betrachtenden, Strahlung. Typischerweise beträgt die Menge an lumineszierenden Substanz 0.01 - 5 Gew.-%, insbesondere 0.1 - 3 Gew.-%, bevorzugt 0.2 - 1 Gew.-%, an der gesamten zweikomponentigen Zusammensetzung.

Bevorzugt enthält die Zusammensetzung mindestens einen Füllstoff. Als Füllstoffe gelten insbesondere anorganische und organische Füllstoffe, zum Beispiel gemahlene oder gefällte Calciumcarbonate, welche gegebenenfalls mit Fettsäuren, insbesondere Stearaten, beschichtet sind, Baryt (BaSO₄, auch Schwerspat genannt), Quarzmehle, calcinierte Kaoline, Aluminiumoxide, Aluminiumhydroxide, Kieselsäuren, insbesondere hochdisperse Kieselsäuren aus Pyrolyseprozessen, Russe, insbesondere industriell hergestellte Russe (im Folgenden als "Russ" bezeichnet), PVC-Pulver oder Hohlkugeln. Typischerweise werden Füllstoffe in der Zusammensetzung in einer Menge von 10 - 60 Gew.-% eingesetzt.

Die Zusammensetzung kann weitere zusätzliche Bestandteile aufweisen. Derartige zusätzliche Bestandteile sind Lösungsmittel, Core-Shell-Polymere, Flüssigkautschuke, organische und anorganische Füllstoffe, Farbstoffe, Pigmente, Inhibitoren, UV- und Hitzestabilisatoren, Antistatika, Flammschutzmittel, Biozide, Weichmacher, Wachse, Verlaufsmittel, Polymerisationsinhibitoren, Haftvermittler, Thixotropiermittel und weitere dem Fachmann bekannte gängige Rohstoffe und Additive.

Es ist dem Fachmann klar, dass er bei der Auswahl der möglichen Bestandteile der Komponenten **K1** und **K2** berücksichtigt, dass diese zusätzlichen Bestandteile nicht mit den bereits vorhandenen Bestandteilen reagiert bzw. die Lagerbeständigkeit der jeweiligen Komponente nicht übermässig verschlechtert.

Das Verhältnis der zwei Komponenten **K1** und **K2** zueinander ist üblicherweise derart gestaltet, dass es möglichst nahe an 1 ist. Bevorzugt wird in den ersten zwei Ausführungsformen ein Volumenverhältnis von **K1** zu **K2** von 5:1 bis 1:1, bevorzugt 3:1 bis 1:1. In der vorgängig beschriebenen dritten Ausführungsform ist aufgrund der Tatsache, dass die Verbindung **X** eine Polymerisation der härtbaren Verbindung **HV** der Komponente **K1** auslöst, sind in der dritten Ausführungsform grössere Mischverhältnisse von Komponente **K1** zu **K2** üblich. Typischerweise beträgt hier das Volumenverhältnis von **K1** zu **K2** von 50:1 bis 1:1, insbesondere 20:1 bis 1:1, bevorzugt 15:1 bis 1:1, meist bevorzugt 12:1 bis 8:1.

Die Zusammensetzung, beziehungsweise die Komponenten, werden vorzugsweise unter Ausschluss von Luftfeuchtigkeit nach üblichen Verfahren gemischt und abgepackt. Feuchtigkeitsempfindliche Komponenten werden vorzugsweise in feuchtigkeitsdichten Verpackungen abgepackt und bis zur Anwendung gelagert. Es ist vorteilhaft und gewünscht, dass während der Herstellung und Lagerung die Komponenten nicht ungewollt reagieren und zu einem Anstieg der Viskosität führen, welcher eine Applikation der Komponente beziehungsweise der Zusammensetzung erschwert oder gar verunmöglicht.

Durch Mischen der zwei Komponenten **K1** und **K2** reagieren die reaktiven Bestandteile, was zu einer Vernetzung bzw. Aushärtung der Zusammensetzung führt.

Die Vermischung der zwei Komponenten kann manuell oder maschinell erfolgen. Ungefüllte Systeme oder leicht pastöse Systeme können einfach mit Rührer oder Mischgeräten wie 2K-Kartuschenpistolen oder mit Pumpen in Kombination mit Statikmischern oder dynamischen Mischern gemischt werden. Hochgefüllte Systeme werden vorteilhaft mittels Rührer von Hand oder Rührwerk gemischt.

Bevorzugte Beispiele für zweikomponentige Zusammensetzungen sind zweikomponentige Epoxydharz-Zusammensetzungen, zweikomponentige Polyurethan-Zusammensetzungen, zweikomponentige (Meth)acrylat-Zusammensetzungen und Wasserpasten-beschleunigte Polyurethanzusammensetzungen.

Ein weiterer Aspekt der vorliegenden Erfindung betrifft die Verwendung einer vorgängig beschriebenen zweikomponentigen Zusammensetzung als Beschichtung, insbesondere als Haftvermittler-Beschichtung.
Eine Haftvermittler-Beschichtung ist eine Beschichtung, welche auf ein Substrat appliziert wird und die Haftung eines darauf applizierten Klebstoffs, Dichtstoffs oder weiteren Beschichtung verbessert, bzw. erst gar ermöglicht. Insbesondere als derartige Haftvermittler-Beschichtungen gelten solche Haftvermittler-Beschichtungen, welche vom Fachmann als Primer, Haftprimer oder Haftvoranstriche bezeichnet werden.

Derartige Haftvermittler-Beschichtungen weisen insbesondere sogenannte Haftvermittler auf. Bei diesen Haftvermittlern handelt es sich vorteilhaft um Silane oder Titanate. Als "Silane" werden hierbei Organo-Siliziumverbindungen bezeichnet, welche mindestens eine an ein Siliziumatom gebundene Hydroxygruppe, Alkoxygruppe oder Acyloxygruppe sowie mindestens einen über eine Kohlenstoff-Silizium-Bindung an ein Siliziumatom gebundenen organischen Substituenten aufweisen. Als "Titanate" werden hierbei Organo-Titanverbindungen bezeichnet, welche mindestens einen über eine Sauerstoff-Titan-Bindung an das Titanatom gebundenen Substituenten aufweisen.

Ein weiterer Aspekt der vorliegenden Erfindung betrifft die Verwendung einer vorgängig beschriebenen zweikomponentigen Zusammensetzung als Klebstoff oder Dichtstoff.

Ein weiterer, wesentlicher, Aspekt der vorliegenden Erfindung betrifft ein Verfahren zur Bestimmung der Mischgüte einer zweikomponentigen Zusammensetzung, wie sie vorgängig im Detail beschrieben wurde, auf einem Substrat umfassend die Schritte:
a) Bestrahlen des mit der zweikomponentigen Zusammensetzung beschichteten Substrates **S1** mit einer Strahlung der Wellenlänge λ₁ von 240 bis 400 nm;
b) Detektierung der von der lumineszierenden Substanz emittierten Strahlung der Wellenlänge λ₂, insbesondere mittels eines Fotodetektors 21.

Das Verfahren kann in einem manuellen und/oder in einem automatisierten Verfahren durchgeführt werden.

Bevorzugt ist ein optischer Filter 22, welcher insbesondere die Strahlung der Wellenlänge λ₁ absorbiert, zwischen Fotodetektor 21 und zweikomponentiger Zusammensetzung angeordnet.

Ein weiterer Aspekt der vorliegenden Erfindung betrifft ein Verfahren zum Verkleben oder Abdichten. Dieses Verfahren umfasst entweder die Schritte
i) Mischen der zwei Komponenten **K1** und **K2** einer zweikomponentigen Zusammensetzung, wie sie vorgängig beschrieben wurde,
ii) Applikation einer gemischten Zusammensetzung auf ein zu verklebendes oder abzudichtendes Substrat **S1**,
iii) Applikation eines Klebstoffes auf die gemischte Zusammensetzung,
iv) Kontaktieren des Klebstoffs mit einem weiteren zu verklebenden oder abzudichtenden Substrat **S2**
   oder die Schritte
   i') Mischen der zwei Komponenten **K1** und **K2** einer zweikomponentigen Zusammensetzung, wie sie vorgängig beschrieben wurde,
   ii') Applikation einer gemischten Zusammensetzung auf ein zu verklebendes oder abzudichtendes Substrat **S1**,
   iii') Kontaktieren der auf Substrat **S1** befindlichen gemischten Zusammensetzung mit einem weiteren zu verklebenden oder abzudichtenden Substrat **S2.**

Das zweite Substrat **S2** besteht hierbei aus demselben oder unterschiedlichem Material wie das Substrat **S1**.

In der ersten Version, d.h. umfassend Schritte i), ii), iii) und iv) stellt die zweikomponentige Zusammensetzung eine Haftvermittler-Beschichtung dar. In der zweiten Version, d.h. umfassend Schritte i'), ii') und iii') stellt die zweikomponentige Zusammensetzung einen Kleb- oder Dichtstoff dar.

Die Art der Substrate **S1** und **S2** kann sehr unterschiedlich sein. Bevorzugte Substrate sind Kunststoffe, insbesondere thermoplastische Kunststoffe, Gläser, Keramiken, Metalle und deren Legierungen, auf natürlichen Materialien basierende Werkstoffe wie Holz, Spanplatten, sowie Lacke. Als meist bevorzugte Substrate gelten lackierte Untergründe, wie lackierte Metallflansche, Kunststoffe, insbesondere PVC, sowie Glas, insbesondere mit Keramik beschichtetes Glas.

Die Mischung der zwei Komponenten kann über Statik- oder dynamische Mischer erfolgen. Die Verwendung eines dynamischen Mischers hat den Vorteil, dass auf einfache Art und Weise die Mischintensität variiert werden kann, zum Beispiel, wie weiter unten beschrieben, über ein Signal von einer Mischgüten-Detektionsstation verursacht. Der Klebstoffauftrag erfolgt üblicherweise als Klebstoffraupe, vorzugsweise als Dreieckraupe.

Die Förderung und die entsprechend korrigierte Dosierung der zwei Komponenten der zweikomponentigen Zusammensetzung kann über eine 2-Komponenten-Kartuschenpistole, welche manuell, hydraulisch oder pneumatisch betrieben werden kann, über eine 2-Komponenten-Kolbenpumpe/- Dosierer, über eine 2- Komponenten-Zahnradpumpe/-Dosierer erfolgen.

Ein weiterer Aspekt der vorliegenden Erfindung betrifft eine Fertigungslinie 1 für die Herstellung eines Artikels 2, welche stromabwärts aufeinander abfolgend
a) mindestens eine Applikationsstation 3 einer gemischten zweikomponentigen Zusammensetzung, wie sie vorgängig beschrieben worden ist, bestehend aus einer ersten Komponenten **K1** und einer zweiten Komponente **K2,**
b) mindestens eine Mischgüten-Detektionsstation 4, und
c) mindestens eine Fügestation 5 aufweist.

Vorteilhaft besteht zwischen Mischgüten-Detektionsstation 4 und Applikationsstation 3 eine Kommunikationsverbindung 10.

Hierbei wird insbesondere aufgrund eines Signals 12, welches über die Kommunikationsverbindung 10 übermittelt wird, das Verhältnis der Dosierung der Menge der ersten Komponente **K1** zur zweiten Komponente **K2** verändert oder ein Alarm 19 oder die Entnahme des Halbfabrikats 11 ausgelöst.

Falls es sich bei der zweikomponentigen Zusammensetzung um eine Haftvermittler-Beschichtung handelt, ist typischerweise zwischen Mischgüten-Detektionsstation 4 und Fügestation 5 eine Klebstoffapplikationsstation vorhanden.

Somit bildet auch ein industriell verklebter Artikel, welcher nach einem oben beschriebenen Verfahren oder mittels einer oben beschriebenen Fertigungslinie hergestellt wurde, einen weiteren Aspekt der vorliegenden Erfindung.

Dieser Artikel ist insbesondere eine Tür oder ein Fenster oder ein Transportmittel oder ein Anbau- oder Einbaumodul eines Transportmittels. Bevorzugt stellt es ein Automobil dar, dessen Scheibe beispielsweise über das beschriebene Verfahren hergestellt wurde.

Weiter bevorzugt ist dieser Artikel ein Fenster oder eine Tür. Besonders bei grossflächigen Fenstern oder Türen weisen die applizierten Klebstoffraupen eine grosse Länge auf. Durch die Länge bedingt, verstreicht zwischen Anfangspunkt und Endpunkt der Klebstoffraupe eine erhebliche Zeit. Nach dem Applizieren des Klebstoffs muss jedoch noch genügend Zeit für das Fügen sein. Es ist somit vor allem in diesen grossflächigen Klebeanwendungen kritisch, dass die Offenzeit an gewissen Orten in der Klebstoffraupe überschritten wird. Ein Überschreiten der Offenzeit bewirkt, dass an diesen Stellen der Klebverbund nicht gewährleistet ist und somit an diesen Stellen Schwachstellen in der Kraftübertragung und/oder Abdichtung vorhanden sind.

Weiterhin können derartige Artikel Artikel der Haushaltsgerätebranche, insbesondere Weisswaren, wie beispielsweise Waschmaschinen oder Geschirrspüler, oder der Möbelbranche sein.

Weitere mögliche Artikel sind Transportgeräte, insbesondere Fahrstühle und Rolltreppen.

Die beschriebenen industriell verklebten Artikel können gelagert und transportiert werden. Aufgrund des Trends von der Fertigung weg vom Band hin ins Zulieferwerk wird die Verwendung von An- und Einbaumodule, welche an einem anderen Ort als die Endfertigung hergestellt werden, zunehmend wichtiger.

Somit bildet ein Bauwerk oder Transportmittel, welches einen wie oben beschriebenen industriell verklebten Artikel aufweist, einen weiteren Aspekt der Erfindung.

Wesentliche Vorteile der vorliegenden Erfindung sind wie folgt: Es lässt sich einerseits grundsätzlich die Anwesenheit derjenigen Komponente, welche die lumineszierende Substanz enthält, nachweisen. Dies ist auch dann der Fall, wenn die beide Komponenten die gleiche Farbe aufweisen oder nur schwach kontrastieren. Weiterhin ist diese Methode auch geeignet für Zusammensetzungen die schwarz ist, weil sie Schwarzpigmente, wie Russ enthalten. Ebenso kann diese Methode auch verwendet werden für Zusammensetzungen die weiss sind, weil sie Weisspigmente, wie Kreide enthalten. Es lässt sich somit zweifelsfrei feststellen, ob eine Komponente zugemischt wird oder nicht. Darüber hinaus lassen sich aber aufgrund bekannter Konzentration der lumineszierenden Substanz in der jeweiligen Komponente und unter Mithilfe einer entsprechenden Eichgerade auch quantitative Aussagen über die Menge der zudosierten Komponente und damit über das Mischungsverhältnis machen und gegebenenfalls das Mischverhältnis korrigieren. Schliesslich kann mit Hilfe einer Bildanalyse Information über die Mischgüte erhalten werden, was wiederum Anlass zur Korrektur, wie Auswechseln des Mischers, sein kann.

Somit kann dank vorliegender Erfindung einer Steigerung der Qualität im Verarbeiten von zweikomponentigen Zusammensetzungen erfolgen.

### Beschreibung der Zeichnungen

Im Folgenden werden anhand der Zeichnungen ausgewählte Ausführungsbeispiele der Erfindung näher erläutert. Gleiche Elemente sind in den verschiedenen Figuren mit den gleichen Bezugszeichen versehen.

### Es zeigen:

- Figur 1: einen schematischen Querschnitt durch ein beschichtetes Substrat beim Verfahren zur Bestimmung der Mischgüte;
- Figur 2: eine perspektivische, schematische Darstellung des Verfahrens zur Bestimmung der Mischgüte am Beispiel einer Automobilverglasung;
- Figur 3: eine schematische Querschnittsdarstellung durch eine Fertigungslinie;
- Figur 4a: Fotographie von Primern mit unterschiedlichen Konzentrationen an Tinopal® SK Slurry unter Bestrahlung mit UV-Licht;
- Figur 4b: eine grafische Darstellung der Abhängigkeit der Intensität der Licht-Emission als Funktion der Konzentration der lumineszierenden Substanz in der Zusammensetzung;
- Figur 5a: Fotographie unter UV-Licht (links) bzw. Tageslicht (rechts) eines (Meth)acrylatklebstoffes ohne lumineszierende Substanz;
- Figur 6a: Fotographie unter UV-Licht (links) bzw. Tageslicht (rechts) eines gut gemischten (Meth)acrylatklebstoffes mit lumineszierender Substanz;
- Figur 6b: Fotographie unter UV-Licht (oben) bzw. Tageslicht (unten) eines gut gemischten (Meth)acrylatklebstoffes mit lumineszierender Substanz;
- Figur 7a: Fotographie unter UV-Licht (links) bzw. Tageslicht (rechts) eines schlecht gemischten (Meth)acrylatklebstoffes mit lumineszierender Substanz;
- Figur 7b: Fotographie unter UV-Licht (oben) bzw. Tageslicht (unten) eines schlecht gemischten (Meth)acrylatklebstoffes mit lumineszierender Substanz.

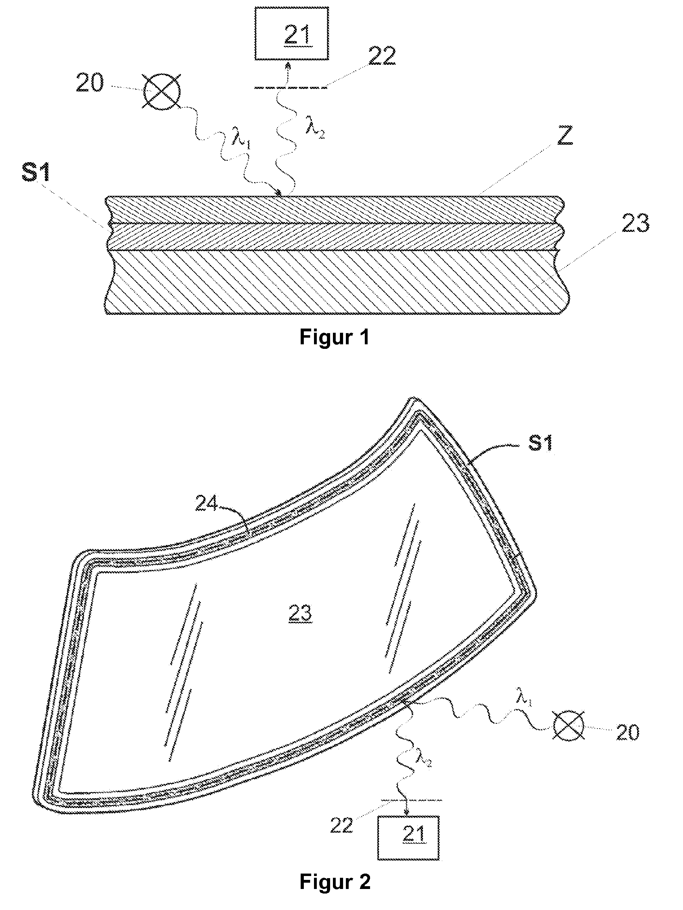

Figur 1 zeigt schematisch einen Querschnitt durch ein Substrat **S1**, welches sich auf einem Trägermaterial 23 befindet und mit einer gemischten zweikomponentigen Zusammensetzung Z beschichtet ist. An diesem Substrat **S1** wird nun ein Verfahren zur Bestimmung der Mischgüte durchgeführt, wobei das Substrat **S1** mittels einer Strahlungsquelle 20 mit einer im UV-Bereich liegenden Strahlung der Wellenlänge λ₁ bestrahlt wird. Durch Anwesenheit einer lumineszierenden Substanz in der zweikomponentigen Zusammensetzung Z emittiert diese eine Strahlung der Wellenlänge λ₂, welche üblicherweise im sichtbaren Lichtbereich liegt und von Auge oder mittels eines Fotodetektors 21 detektiert werden kann. Optional kann dem Fotodetektor ein optischer Filter 22 vorgelagert werden, der insbesondere die Strahlung der Strahlungsquelle 20 mit der Wellenlänge λ₁ vor der Detektierung herausfiltert.

Durch das Verfahren, wie es in Figur 1 dargestellt ist, kann einerseits sichtbar gemacht werden, dass diejenige Komponente, welche die lumineszierende Substanz enthält aufgebracht wurde und aufgrund der Bildanalyse, beispielsweise mittels einer Bildauswertung auf einem Computer (hier nicht dargestellt), wie gut die zwei Komponenten gemischt wurden.

Figur 2 zeigt eine perspektivische schematische Darstellung einer bevorzugten Anwendung des in Figur 1 dargestellten Verfahrens. Dabei wird eine Automobilverglasung 24, deren Glaskeramikrand in diesem Fall das Substrat **S1** darstellt und mit der gemischten zweikomponentigen Zusammensetzung in der Form einer Haftvermittler-Beschichtung oder Klebstoffs Z beschichtet ist, mittels einer Strahlungsquelle 20 mit einer Strahlung der Wellenlänge λ₁ bestrahlt. Durch die Anwesenheit einer lumineszierenden Substanz in der Zusammensetzung Z emittiert diese wiederum eine Strahlung der Wellenlänge λ₂, welche von Auge oder mittels eines Fotodetektors 21 detektiert werden kann. Wie bereits in Figur 1 dargestellt, kann es nützlich sein, dem Fotodetektor 21 einen optischen Filter 22 vorzulagern.

Durch die Anwendung des Verfahrens, wie in Figur 2 dargestellt, kann einerseits überprüft werden, ob die Zusammensetzung am dafür vorhergesehen Ort auf dem Substrat **S1**, in diesem Fall durchgehend auf dem Randbereich der Scheibe, appliziert worden ist und somit eine optimale Haftung gewährleistet werden kann. Andererseits kann bei einem unerwünschten Verlust der Hafteigenschaften überprüft werden, ob die vorgeschriebene Haftvermittlerzusammensetzung wie vorgesehen angewendet wurde.

Das Verfahren, wie es hier vor allem in Figur 2 beschrieben wird, kann optional teilweise oder vollumfänglich automatisiert durchgeführt werden. Bevorzugt wird dies in Fällen, wo das Verfahren dazu verwendet wird, um zu überprüfen, ob die Zusammensetzung am dafür vorhergesehenen Ort auf dem Substrat **S1**, im Fall der Figur 2 durchgehend auf dem Randbereich der Fahrzeugscheibe, appliziert worden ist. Dabei können der Auftrag der gemischten zweikomponentigen Zusammensetzung Z, die Detektierung und der gegebenenfalls (d.h., wenn die zweikomponentige Zusammensetzung eine Haftvermittler-Beschichtung ist) anschliessende Auftrag des Kleb- oder Dichtstoffes unmittelbar nacheinander erfolgen. Unterstützend kann dabei geeignete Software zur Bilderfassung und/oder zur Robotersteuerung beim Auftrag der Haftvermittler-Beschichtung und/oder des Kleb- oder Dichtstoffes zum Einsatz kommen.

Bevorzugt kann das Verfahren während der Offenzeit der Zusammensetzung Z noch vor dem Auftragen des Kleb- oder Dichtstoffes bzw. vor dem Fügen der Scheibe durchgeführt werden. Dadurch ist es möglich Substrate, wie Fahrzeugscheiben, die einen mangelhafte Applikation der Zusammensetzung Z erhalten haben, innerhalb eines Linienherstellungsprozesses zu erkennen und auszuschliessen, bevor der Klebstoff darauf appliziert, bzw. der Klebverbund erstellt wird. Dies ist besonders vorteilhaft, weil dadurch die unnötige Verschwendung von Klebstoff vermindert werden kann.

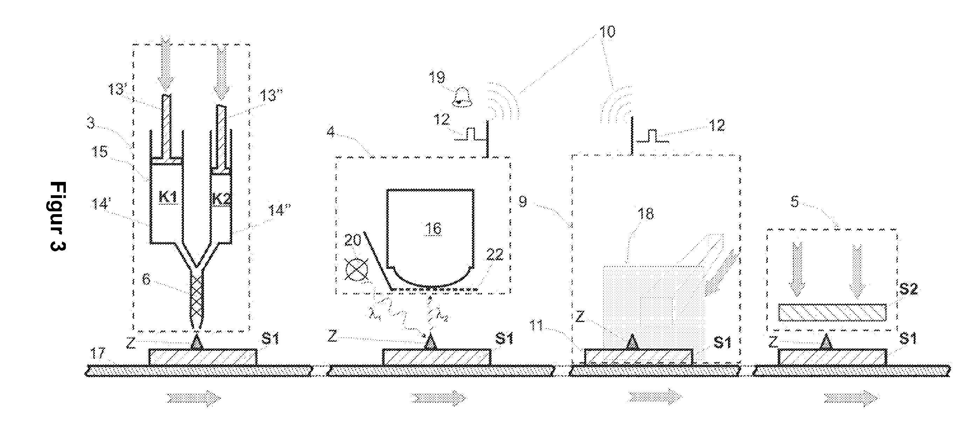

Figur 3 stellt einen schematischen Querschnitt durch eine Fertigungslinie 1 zur Fertigung eines Artikels 2 mit einer Entnahmestation 9 dar. In der hier gewählten Darstellung ist die zweikomponentige Zusammensetzung Z ein Klebstoff.

Die hier dargestellte Fertigungslinie 1 weist ein Förderband 17 auf, auf welchem ein Substrat **S1** zuerst zu einer Klebstoffapplikationsstation 3, anschliessend zu einer Mischgüten-Detektionsstation 4, anschliessend zu einer Entnahmestation 9 und schliesslich zu einer Fügestation 5 bewegt wird. Bei der Klebstoffapplikationsstation 3 wird der über einen Mischer 6 aus den Komponenten **K1** und **K2** gemischte Klebstoff Z auf die Oberfläche des Substrates **S1**, insbesondere als Dreiecksraupe, appliziert. In der hier dargestellten Ausführungsform erfolgt die Dosierung der ersten Komponente **K1**, beziehungsweise der zweiten Komponente **K2,** durch einen Dosierkolben 13' bzw. 13", aus einem Kartuschenzylinder 14', resp. 14". Es ist dem Fachmann klar, dass anstelle der hier gezeigten Variante einer Zwillingskartusche 15, auch andere Förder- bzw. Dosiervorrichtungen verwendet werden können, wie beispielsweise Förderung/Dosierung mittels Kolbenpresse über Folgeplatte aus Hobbocks oder Fässern. Die Bewegung der Dosierkolben 13' und 13" erfolgt bevorzugt getrennt von einander, was die Optimierung der Dosierverhältnisse, bzw. Dosiermengen, erlaubt.

In einer weiteren Station stromabwärts im Herstellprozess eines Artikels wird mittels Strahlungsquelle 20 die lumineszierende Substanz über Licht der Wellenlänge λ₁ angeregt, wodurch diese Licht der Wellenlänge λ₂ emittiert. Dieses Licht wird durch den Fotodetektor 16 erfasst und analysiert. Die Analyse erfolgt in einem Computer (nicht dargestellt). Falls festgestellt wird, dass keine Zusammensetzung, genauer keine lumineszierende Substanz, vorhanden ist, oder aber die Mischgüte schlecht ist, löst der Computer ein Signal 12 aus, welches einen Alarm 19 auslöst oder über eine Kommunikationsverbindung 10 an eine Entnahmestation 9 übermittelt wird. In der hier dargestellten Form ist die Kommunikationsverbindung 10 eine Funkverbindung. Die Kommunikationsverbindung 10 kann jedoch auch ein Kabel sein. Die Kommunikationsverbindung 10 kann sowohl einseitig als auch zweiseitig sein.

In einer weiteren Station im Herstellprozess eines Artikels kommt das Halbfabrikat 11 zur Entnahmestation 9. Diese Entnahmestation entnimmt gegebenenfalls das Halbfabrikat aufgrund eines über die Kommunikationsverbindung 10 übermittelten Signals 12 das Halbfabrikat, welches die Fertigungslinie bis zur Entnahmestation durchlaufen hat, aus der Fertigungslinie. Zur Entnahme besitzt diese Entnahmestation 9 ein Entnahmemittel 18. Dieses Entnahmemittel 18 kann verschieden ausgebildet sein. Es kann sich beispielsweise um einen Robotergreifarm oder, wie in Figur 3 dargestellt, um einen Schieber 18 handeln, welcher das Halbfabrikat durch eine Bewegung quer zur Laufrichtung des Förderbandes 17 aus der Fertigungslinie 1 entfernt.

Falls keine Entnahme aktiviert wird, bleibt das Halbfabrikat in der Fertigungslinie und gelangt zu einer weiteren Station, der Fügestation 5. Dort wird das Halbfabrikat, d.h. das Substrat **S1** mit dem darauf applizierten Klebstoff K, mit einem weiteren Substrat, **S2,** gefügt. Nach dem Fügen erfolgt die endgültige Vernetzung und es resultiert ein verklebter Artikel 2.

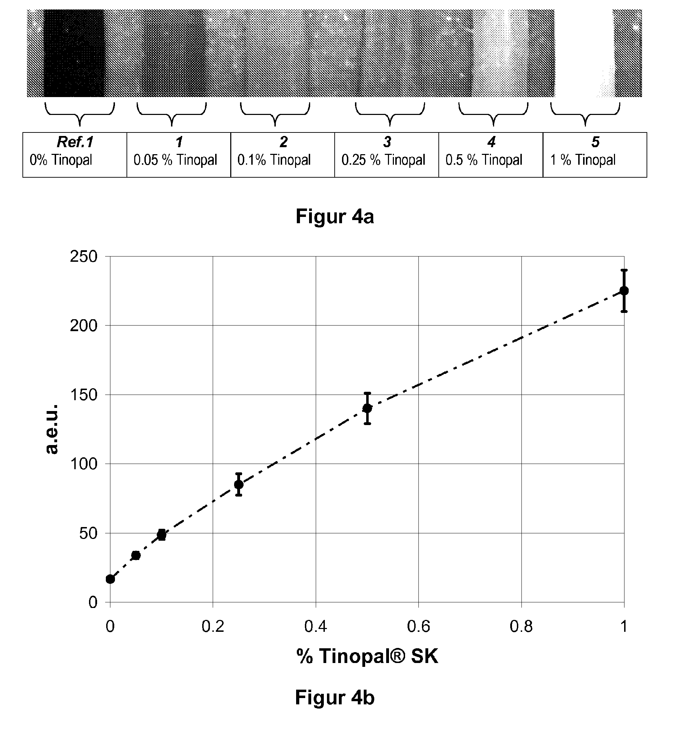

Für die Figuren 4a und 4b wurden gemäss Tabelle 1 Formulierungen einer wässrigen, zweikomponentigen Haftvermittler-BeschichtungsZusammensetzung mit unterschiedlichen Konzentrationen an Tinopal® SK Slurry, kommerziell erhältlich von Ciba Specialty Chemicals, hergestellt.

**Tabelle 1. Zusammensetzungen mit unterschiedlicher Menge an lumineszierender Substanz. (Mengen in Gew.% bez. auf Zusammensetzung)**

| **K1** | ***Ref.1*** | ***1*** | ***2*** | ***3*** | ***4*** | ***5*** |
|---|---|---|---|---|---|---|
| Beckopox® EP 384 w | 53.82 | 53.82 | 53.82 | 53.82 | 53.82 | 53.82 |
| Cytec | | | | | | |
| Tinopal® SK Slurry | | 0.05 | 0.1 | 0.25 | 0.5 | 1 |
| | | | | | | |

| **K2** | | | | | | |
|---|---|---|---|---|---|---|
| Beckopox®VEH 2188w | 20.87 | 20.87 | 20.87 | 20.87 | 20.87 | 20.87 |
| Cytec | | | | | | |
| Russ | 6 | 6 | 6 | 6 | 6 | 6 |
| Wasser | 19.31 | 19.26 | 19.21 | 19.06 | 18.81 | 18.31 |
| ***K1* + *K2*** | 100 | 100 | 100 | 100 | 100 | 100 |

Die zwei Komponenten wurden gemischt und auf eine Glasplatte mittels getränktem Schwamm appliziert.

Darauf folgend wurde die Probe mit UV-Licht der Wellenlänge zwischen 360 und 380 nm bestrahlt und mit einer Nikon E4300 Digitalkamera fotografiert. Die entstandene Digital-Fotografie mit den Beispielen ***Ref.1*** und ***1*** bis ***5*** wurde mit Hilfe der Bildbearbeitungs-Software Corel® Photo-Paint 11.0 der Firma Corel® UK Limited in Graustufen (8 bit) umgewandelt und in Figur 4a aufgezeigt.

Figur 4b zeigt eine grafische Darstellung der Abhängigkeit der Intensität der Licht-Emission als Funktion der Konzentration der lumineszierenden Substanz in den Beispielen ***Ref.1*** und ***1*** bis ***5.*** Dazu wurde der oben beschriebene Glas-Prüfkörper mit den Bahnen der obigen Haftvermittler-Beschichtungs-Zusammensetzungen mittels Mikroskop untersucht. Die Probe wurde unter dem Mikroskop (Olympus BX51 M, Objektiv 5x, Kameraaufsatz zu CCD-Kamera mit 2048x1536 Bildpunkten) mittels einer Hg-Dampflampe mit UV-Licht der Wellenlängen 420 - 440 nm bestrahlt und die Emission bei Wellenlängen > 475 nm beobachtet. Von den Bildern der entsprechenden Haftvermittlerzusammensetzungsbahnen wurden jeweils Ausschnitte von ca. 0.4 x 0.4 mm für die quantitative Analyse verwendet. Der durchschnittliche Intensitätswert des Blaukanals für einen solchen Bildausschnitt wurde mittels Bildanalyse ermittelt. Aus fünf Bildern mit jeweils neun solcher Ausschnitte wurden pro Konzentration so 45 Intensitätswerte des Blaukanals gemittelt und die Standardabweichung dieser Werte bestimmt. Der mittlere Intensitätswert (a.e.u. = aribtrary emmision unit) des Blaukanals einer Probe wurde gegen deren jeweilige Tinopal® SK Slurry-Konzentration aufgetragen. Aufgrund dieser Eichkurve und entsprechend gemessener Emission lassen sich somit auch unbekannte Konzentrationen bestimmen.

Die Ergebnisse dieser in Figur 4a und 4b visualisierten Versuche zeigen, dass Zusammensetzungen, auch wenn sie mit einem Schwarzpigment, nämlich mit Russ, gefüllt sind, sich auf einfache Art und Weise nachweisen, beziehungsweise unterscheiden, lassen, und sich daraus auch die Menge der jeweilig eingesetzten Komponenten, bzw. die Mischverhältnisse, bestimmen lassen.

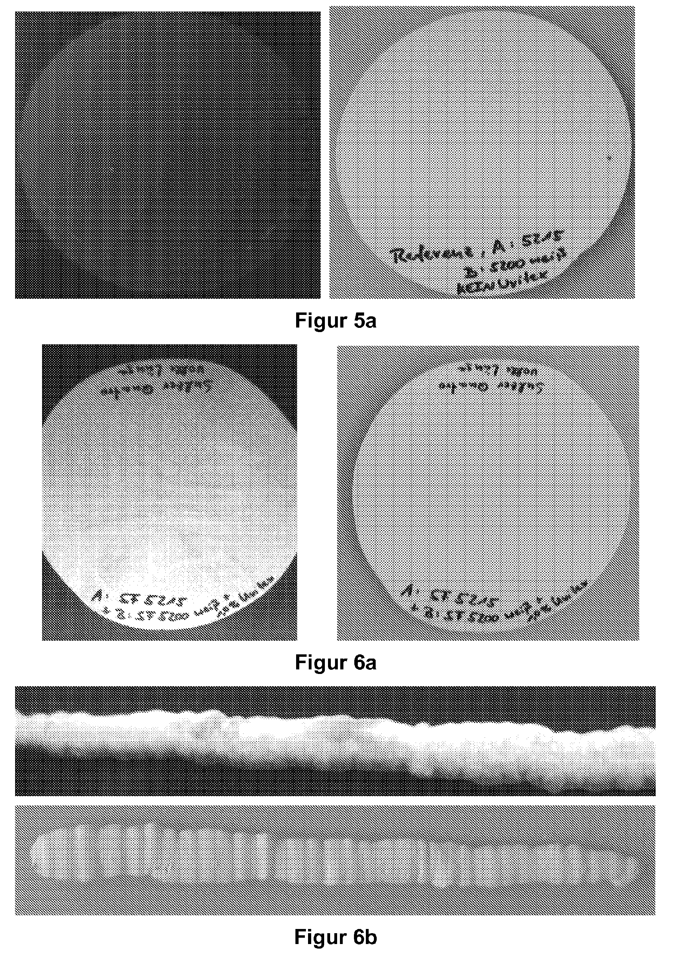

Figuren 5a) bis 7b) zeigen ein Bespiel, bei welchem die Mischgüte einer zweikomponentigen Zusammensetzung bestimmt wurde.

Hierfür wurde ein (Meth)acrylatklebstoff wie folgt verwendet. Als erste Komponente, welche das (Meth)acrylatmonomer enthält, wurde die A-Komponente des bei Sika Schweiz AG kommerziell erhältlichen SikaFast®-5215 verwendet und im Vergleichsbeispiel ***Ref.2*** mit der weissen B-Komponente des bei Sika Schweiz AG kommerziell erhältlichen SikaFast®-5200 kombiniert, welche ein organisches Peroxid enthält. Für das Beispiel ***6*** wurde als B-Komponente ebenfalls die weisse B-Komponente des SikaFast®-5200 verwendet, der nun aber 1 % bzw. 0.1 % Uvitex® OB, kommerziell erhältlich von Ciba Specialty Chemicals, in einem Speedmixer zugemischt wurde.

Es wurden nun die A- und B-Komponente in die Kammern einer Dualkartusche gefüllt und im Volumenverhältnis A:B =10:1 appliziert.

Die Mischung erfolgte über einen Sulzer-Quadro-Mischer mit 12 bzw. mit 4 Mischelementen. So wurden dann einerseits Raupen und andererseits "Felle" erstellt. Für die "Felle" wurde der Klebstoff auf eine Polyethylenplatte appliziert und mit einer zweiten Polyethylenplatte verpresst. Die Dicke des Felles wurde mittels Abstandshalter zwischen den Polyethylenplatten auf 2 mm eingestellt. Nach einer Wartenzeit von 15 Minuten war der Klebstoff ausgehärtet und die Polyethylen-Deckplatte wurde entfernt.

Es wurden nun die Raupen, bzw. Felle bei Tageslicht, bzw. UV-Licht, (Hg-Dampflampe: Wellenlängen 420 - 440 nm) betrachtet und fotografiert. In den Figuren 5a) bis 7b) sind diese in Graustufen, wie für Fig. 4a) beschrieben, umgewandelten Aufnahmen aufgeführt.
Fig. 5a) zeigt links die Aufnahme unter UV-Licht und rechts bei Tageslicht des Felles des Vergleichsbeispiels ***Ref.2*** (d.h. ohne lumineszierende Substanz) gemischt mit 12 Mischelementen.
Fig. 6a) zeigt links die Aufnahme unter UV-Licht und rechts bei Tageslicht des Felles des Beispiels ***6*** gemischt mit 12 Mischelementen.
Fig. 6b) zeigt oben die Aufnahme unter UV-Licht und unten bei Tageslicht einer Raupe des Beispiels ***6*** gemischt mit 12 Mischelementen.

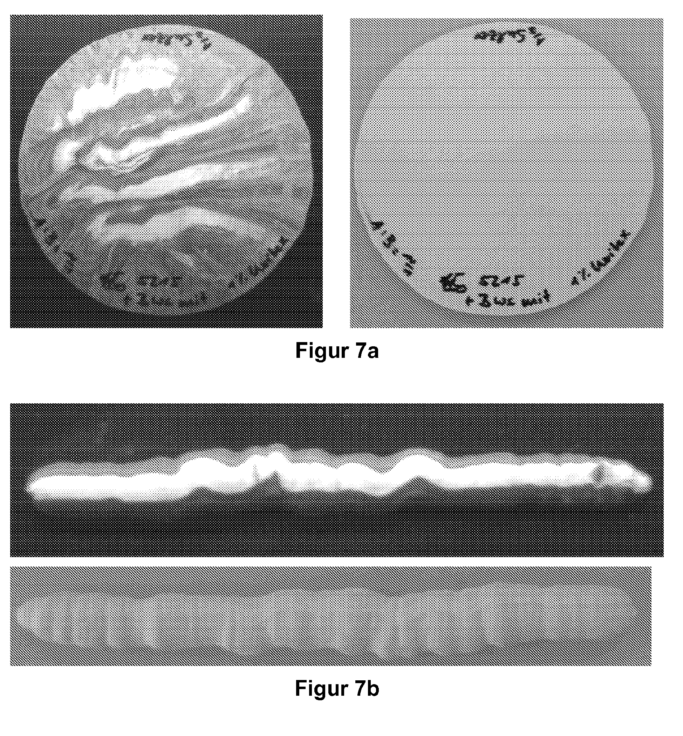

Fig. 7a) zeigt links die Aufnahme unter UV-Licht und rechts bei Tageslicht des Felles des Beispiels ***6*** gemischt mit lediglich 4 Mischelementen.
Fig. 7b) zeigt oben die Aufnahme unter UV-Licht und unten bei Tageslicht einer Raupe des Beispiels ***6*** gemischt mit lediglich 4 Mischelementen.

Aus den Figuren 5a) bis 7b) ist einerseits ersichtlich, dass bei Tageslicht alle Proben gleich aussehen und alle homogen vermischt zu sein scheinen. Es sind somit visuell keine Unterschiede ersichtlich, ob eine lumineszierende Substanz vorhanden ist, bzw. wie viele Mischelemente im Statikmischer vorhanden waren.

Bei Betrachtung unter UV-Licht hingegen lassen sich klare Unterschiede in der Mischgüte erkennen. Bei lediglich 4 Mischelementen sind Schlieren- bzw. Marmorierungseffekte auf dem Fell (Fig. 7a, links) erkennbar, während bei 12 Mischelementen eine homogene Mischung vorhanden ist (Fig. 6a, links). Auch bei den Raupen lassen sich unter UV-Licht bei der schlechteren Mischung (Fig. 7b, oben) deutliche Schlieren, bzw. Schichten, feststellen, während bei der guten Mischung (Fig. 6b oben) dies nicht der Fall ist. Dort ist die Mischung homogen.

Werden nun aus den Fellen Probenkörper an unterschiedlichen Orten herausgestanzt und deren Zugfestigkeit gemessen, stellt sich heraus, dass beim Beispiel der guten homogenen Mischung (Fig. 6a) bedeutend höhere Werte und mit massiv engerer Streuung gemessen werden, als beim entsprechenden Beispiel mit schlechterer Mischung (Fig. 7a).

Somit lassen sich mit dieser Methode Schwachstellen in der Mischgüte erkennen, die bei Betrachtung unter Tageslicht, nicht erkennbar sind.

Entsprechendes Verhalten wurde auch bei der Kombination von A-Komponente des SikaFast®-5215 mit der schwarzen B-Komponente des SikaFast®-5215, welche ein organisches Peroxid enthält, gefunden.

### Bezugszeichenliste

- 1: Fertigungslinie
- 2: Artikel
- 3: Applikationsstation
- 4: Mischgüten-Detektionsstation
- 5: Fügestation
- 6: Mischer
- 9: Entnahmestation
- 10: Kommunikationsverbindung
- 11: Halbfabrikat
- 12: Signal
- 13', 13": Dosierkolben
- 14', 14": Kartuschenzylinder
- 15: Zwillingskartusche
- 16: Fotodetektor
- 17: Förderband
- 18: Entnahmemittel, Schieber
- 19: Alarm
- 20: Strahlungsquelle
- 21: Fotodetektor
- 22: optischer Filter
- 23: Trägermaterial
- 24: Automobilverglasung
- λ₁: Strahlung mit Anregungs-Wellenlänge
- λ₂: Strahlung mit Emissions- Wellenlänge
- Z: Gemischte zweikomponentige Zusammensetzung enthaltend lumineszierende Substanz
- **K1**: erste Komponente
- **K2**: zweite Komponente
- **S1**: erstes Substrat
- **S2**: zweites Substrat

## Patentansprüche

1. Zweikomponentige Zusammensetzung bestehend aus einer ersten Komponenten **K1** und einer zweiten Komponente **K2,** wobei die Komponente **K1** mindestens eine härtbare Verbindung **HV** enthält,
und
die Komponente **K2**
mindestens eine Verbindung **X** enthält, welche mit der härtbaren Verbindung **HV** der Komponente **K1** reaktionsfähig ist oder welche die Polymerisation der härtbaren Verbindung **HV** der Komponente **K1** auslöst, **dadurch gekennzeichnet, dass** eine der zwei Komponenten **K1** oder **K2** mindestens eine lumineszierende Substanz **L** enthält.

2. Zweikomponentige Zusammensetzung gemäss Anspruch 1, **dadurch gekennzeichnet, dass** die härtbare Verbindung **HV** ein Polyepoxid ist und die Verbindung **X** ausgewählt ist aus der Gruppe bestehend aus Polyaminen, Polymercaptanen, Polyamidoaminen, (Poly)Aminoalkoholen, (Poly)Mercaptoalkoholen und (Poly)Aminomercaptanen.

3. Zweikomponentige Zusammensetzung gemäss Anspruch 1, **dadurch gekennzeichnet, dass** die härtbare Verbindung **HV** ein Polyisocyanat ist und die Verbindung **X** ausgewählt ist aus der Gruppe bestehend aus Wasser, Polyolen, Polyaminen, Polymercaptanen, Polyamidoaminen, (Poly)-Aminoalkoholen, (Poly)Mercaptoalkoholen und (Poly)Aminomercaptanen.

4. Zweikomponentige Zusammensetzung gemäss Anspruch 1, **dadurch gekennzeichnet, dass** die härtbare Verbindung **HV** ein olefinisch ungesättigtes Monomer und/oder Oligomer, insbesondere welches ausgewählt ist aus der Gruppe bestehend aus Styrol, Acrylonitril, (Meth)acrylamide, (Meth)acrylsäure, (Meth)acrylate, Vinylalkohole, Vinylether, Vinylester, und ungesättigte Polyester, bevorzugt ein (Meth)acrylate, ist; und die Verbindung **X** ein Radikalbildner, insbesondere eine Perester oder ein organisches Peroxid oder ein Hydroperoxid, bevorzugt ein organisches Peroxid, ist.

5. Zweikomponentige Zusammensetzung gemäss einem der vorhergehenden Ansprüche, **dadurch gekennzeichnet, dass** die lumineszierende Substanz **L** ausgewählt ist aus der Gruppe bestehend aus Stilben-Derivate, Pyrazoline, Bis(benzoxazol-2-yl)-Derivate und Cumarine.

6. Zweikomponentige Zusammensetzung gemäss einem der vorhergehenden Ansprüche, **dadurch gekennzeichnet, dass** die Menge an lumineszierender Substanz **L** 0.01 - 5 Gew.-%, insbesondere 0.1 - 3 Gew.-%, bevorzugt 0.2 - 1 Gew.-%, an der gesamten zweikomponentigen Zusammensetzung beträgt.

7. Verwendung einer zweikomponentigen Zusammensetzung gemäss einem der Ansprüche 1 bis 6 als Beschichtung, insbesondere als Haftvermittler.

8. Verwendung einer zweikomponentigen Zusammensetzung gemäss einem der Ansprüche 1 bis 6 als Klebstoff oder Dichtstoff.

9. Verfahren zur Bestimmung der Mischgüte einer zweikomponentigen Zusammensetzung gemäss einem der Ansprüche 1 bis 6 auf einem Substrat umfassend die Schritte
a) Bestrahlen des mit der zweikomponentigen Zusammensetzung beschichteten Substrates **(S1)** mit einer Strahlung der Wellenlänge λ₁ von 240 bis 400 nm;
b) Detektierung der von der lumineszierenden Substanz emittierten Strahlung der Wellenlänge λ₂, insbesondere mittels eines Fotodetektors (21).

10. Verfahren nach Anspruch 9, **dadurch gekennzeichnet, dass** ein optischer Filter (22) zwischen Fotodetektor (21) und zweikomponentiger Zusammensetzung angeordnet ist, welcher insbesondere die Strahlung der Wellenlänge λ₁ absorbiert.

11. Verfahren zum Verkleben oder Abdichten umfassend entweder die Schritte
i) Mischen der zwei Komponenten **K1** und **K2** einer zweikomponentigen Zusammensetzung gemäss einem der Ansprüche 1 bis 6,
ii) Applikation einer gemischten Zusammensetzung auf ein zu verklebendes oder abzudichtendes Substrat **S1**,
iii) Applikation eines Klebstoffes auf die gemischte Zusammensetzung,
iv) Kontaktieren des Klebstoffs mit einem weiteren zu verklebenden oder abzudichtenden Substrat **S2**
oder die Schritte
i') Mischen der zwei Komponenten **K1** und **K2** einer zweikomponentigen Zusammensetzung gemäss einem der Ansprüche 1 bis 6,
ii') Applikation einer gemischten Zusammensetzung auf ein zu verklebendes oder abzudichtendes Substrat **S1**,
iii') Kontaktieren der auf Substrat **S1** befindlichen gemischten Zusammensetzung mit einem weiteren zu verklebenden oder abzudichtenden Substrat **S2,**
wobei das zweite Substrat **S2** aus demselben oder unterschiedlichem Material wie das Substrat **S1** besteht.

12. Fertigungslinie (1) für die Herstellung eines Artikels (2), welche stromabwärts aufeinander abfolgend
a) mindestens eine Applikationsstation (3) einer gemischten zweikomponentigen Zusammensetzung gemäss einem der Ansprüche 1 bis 6, bestehend aus einer ersten Komponenten **K1** und einer zweiten Komponente **K2,**
b) mindestens eine Mischgüten-Detektionsstation (4), und
c) mindestens eine Fügestation (5) aufweist.

13. Fertigungslinie gemäss Anspruch 12, **dadurch gekennzeichnet, dass** zwischen Mischgüten-Detektionsstation (4) und Applikationsstation (3) eine Kommunikationsverbindung (10) besteht.

14. Fertigungslinie gemäss Anspruch 13, **dadurch gekennzeichnet, dass** aufgrund eines Signals (12), welches über die Kommunikationsverbindung (10) übermittelt wird, das Verhältnis der Dosierung der Menge der ersten Komponente **K1** zur zweiten Komponente **K2** verändert wird oder ein Alarm (19) oder die Entnahme des Halbfabrikats (11) ausgelöst wird.

15. Fertigungslinie gemäss Anspruch 14, **dadurch gekennzeichnet, dass** zwischen Mischgüten-Detektionsstation (4) und Fügestation (5) eine Klebstoffapplikationsstation vorhanden ist.

16. Industriell verklebter Artikel, welcher nach einem Verfahren gemäss Anspruch 11 oder mittels einer Fertigungslinie gemäss einem der Ansprüche 12 bis 15 hergestellt wird.

17. Industriell verklebter Artikel gemäss Anspruch 16, **dadurch gekennzeichnet, dass** der Artikel eine Tür oder ein Fenster oder ein Transportmittel oder ein An- oder Einbaumodul eines Transportmittels ist.

18. Bauwerk oder Transportmittel aufweisend einen industriell verklebten Artikel gemäss Anspruch 16 oder 17.
